(19) Europäisches Patentamt
European Patent Office
Office européen des brevets

(11) Publication number: **0 145 207**
**B1**

(12) **EUROPEAN PATENT SPECIFICATION**

(45) Date of publication of patent specification: **25.01.89**

(51) Int. Cl.$^4$: **C 07 D 473/18,**
C 07 C 43/174, C 07 C 43/192,
C 07 C 69/12, A 61 K 31/52

(21) Application number: **84307373.5**

(22) Date of filing: **26.10.84**

(54) **Purine derivatives.**

(30) Priority: **31.10.83 US 547297**
**05.10.84 US 657211**

(43) Date of publication of application:
**19.06.85 Bulletin 85/25**

(45) Publication of the grant of the patent:
**25.01.89 Bulletin 89/04**

(84) Designated Contracting States:
**AT BE CH DE FR GB IT LI LU NL SE**

(56) References cited:
**EP-A-0 049 072**
**CH-A- 623 587**
**DE-A-3 234 610**
**GB-A-1 567 671**

**JOURNAL OF MEDICINAL CHEMISTRY, vol. 26, no. 5, May 1983 J.C. MARTIN et al. "9-(1,3-Dihydroxy-2-propoxy) methyl) guanine: A New Patent and Selective Antiherpes Agent" pages 759-761**

**BIOCHEMICAL PHARMACOLOGY, vol. 30, no. 13, July 1, 1981 P.M. KELLER et al. "Enzymatic phosphorylation of acyclic nucleoside analogs and correlations with antiherpetic activities" pages 3071-3077**

(73) Proprietor: **WARNER-LAMBERT COMPANY**
**201 Tabor Road**
**Morris Plains New Jersey 07950 (US)**

(72) Inventor: **Sircar, Jagadish C.**
**3615 Charter Place**
**Ann Arbor Michigan 48104 (US)**
Inventor: **Schwender, Charles F.**
**6455 Walsh Road**
**Dexter Michigan 48130 (US)**
Inventor: **Suto, Mark J.**
**3410 Brentwood Court**
**Ann Arbor Michigan 48104 (US)**

(74) Representative: **Jones, Michael Raymond et al**
**HASELTINE LAKE & CO. Hazlitt House 28**
**Southampton Buildings Chancery Lane**
**London WC2A 1AT (GB)**

(56) References cited:

**CANADIAN JOURNAL OF CHEMISTRY, vol. 60, no. 24, December 15, 1982 K.K. OGILVIE et al. "Biologically active acyclonucleoside analogues. II. The synthesis of 9-(2-hydroxy-1-(hydroxymethyl)-ethoxy)methyl) guanine (BIOLF-62)" pages 3005-3010**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 145 207 B1**

## Description

One aspect of the present invention relates to a compound of the formula

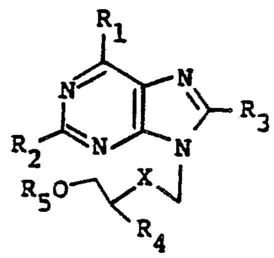

1

wherein $R_1$ is OH or SH; $R_2$ is hydrogen or NHR in which R is hydrogen or $COR_6$ where $R_6$ is alkyl of 1—4 carbon atoms, aryl or aralkyl; $R_3$ is bromine or NHR where R is hydrogen or $COR_6$; X is O or S; $R_4$ is hydrogen or $CH_2OR_5$; and $R_5$ is hydrogen, alkyl of 1—8 carbon atoms, aryl aralkyl,

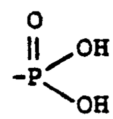

or $COR_6$; with the proviso that, when $R_1$ is —OH, $R_2$ is —$NH_2$, $R_3$ is a bromine atom and $R_5$ is a hydrogen atom, $R_4$ is not a hydrogen atom; or a tautomeric isomer thereof, or a pharmaceutically acceptable acid or base addition salt thereof.

In Biochem. Pharm., *30* (1981) pages 8071—3077 2-amino-8-bromo-9-[(2-hydroxyethoxy)methyl]-9H-purin-6-ol and further structurally related purine derivatives having antiviral activities are disclosed.

European Patent Application No. 0249247 which is a first divisional of the present application, relates to a compound of the formula *1*, wherein $R_1$ is OH or SH; $R_2$ is hydrogen of NHR in which R is hydrogen or $COR_6$ where $R_6$ is alkyl of one to four carbon atoms, aryl or aralkyl; $R_3$ is hydrogen; X is O or S; $R_4$ is alkyl of one to eight carbon atoms, aryl or aralkyl, and $R_5$ is hydrogen, or a tautomeric isomer thereof, or a pharmaceutically acceptable acid or base addition salt thereof.

European Patent Application No. 0249248, which is a second divisional of the present application, relates to a compound of the formula *1*, wherein $R_1$ is OH or SH; $R_2$ is hydrogen or NHR in which R is hydrogen or $COR_6$ where $R_6$ is alkyl of one to four carbon atoms, aryl or aralkyl; $R_3$ is hydrogen; X is O or S; $R_4$ is $CH_2OR_7$ in which $R_7$ is alkyl of one to eight carbon atoms, cycloalkyl of five to seven ring members, cycloalkylalkyl, aryl or arylalky, and $R_5$ is hydrogen, or a tautomeric isomer thereof, or a pharmaceutically acceptable acid or base addition salt thereof.

Other aspects of the invention include a method of manufacture and a pharmaceutical composition comprising an effective amount of a compound according to the present invention with a pharmaceutically acceptable carrier. Compounds of the present invention are useful for treating autoimmune diseases such as arthritis, systemic lupus erythematosus, inflammatory bowel diseases, transplantation, juvenile diabetes, myasthenia gravis, multiple sclerosis as well as viral infections and cancer by administering an effective amount of a compound according to the present invention in unit dosage form.

The term "alkyl of 1—8 carbon atoms" means a straight or branched hydrocarbon chain up to 8 carbon atoms such as, for example, methyl, ethyl, propyl, isopropyl, butyl, isobutyl, sec-butyl, tertiary-butyl, or octyl.

The term "cycloalkyl of five to seven ring members" means cyclopentyl, cyclohexyl, or cycloheptyl.

The term "cycloalkylalkyl" means a cyclopentyl, cyclohexyl, or cyclopheptyl radical attached to an alkyl chain of up to four carbon atoms, straight or branched, such as for example, cyclohexylmethyl or cyclohexylethyl.

The term "aryl" relates to phenyl rings which are unsubstituted and substituted by halo, e.g., fluoro, chloro, bromo, or alkyl of 1—4 carbon atoms, such as methyl or ethyl, hydroxy, alkoxy of 1—4 carbon atoms, such as methoxy or ethoxy, or trifluoromethyl.

The term "aralkyl" relates to an "aryl" radical as defined above attached to an alkyl chain of up to 4 carbon atoms, such as unsubstituted or substituted phenylethyl or benzyl where the substituents on the aromatic ring may be the same as defined above.

Pharmaceutically acceptable base salts of the phosphate ester, where $R_5$ is

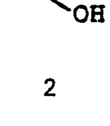

are the alkali metals, ammonium or substituted ammonium salts, such as sodium, potassium, and ammonium salts. The base salts may be prepared by standard methods known in the art.

Pharmaceutically acceptable acid addition salts are those derived from inorganic acids such as hydrochloric, sulfuric and the like, as well as organic acids such as methanesulfonic, toluenesulfonic, tartaric acid, and the like. These salts may also be prepared by standard methods known in the art.

Other pharmaceutically acceptable salts are those derived from inorganic bases such as sodium hydroxide, potassium hydroxide or ammonium hydroxide or organic bases such as arginine, N-methyl glucamine, and the like. These salts may also be prepared by standard methods known in the art.

Preferably $R_6$ isa $C_{1-4}$ alkyl radical or a phenyl radical; $R_3$ is bromine or $NH_2$; and $R_5$ is a hydrogen atom, a $C_{1-8}$ radical, a phenyl radical, or a benzyl radical.

Preferably $R_1$ is —OH; $R_2$ is hydrogen or —$NH_2$; $R_3$ is bromine or —$NH_2$; X is an oxygen atom; $R_4$ is a hydrogen atom or —$CH_2OR_5$; and $R_5$ is a hydrogen atom.

Particular embodiments of the compounds of the present invention include 2,8-diamino-9-[(2-hydroxyethoxy)methyl-9*H*-purin-6-ol, 2-[(2,8-diamino-6-hydroxy-9*H*-purin-9-yl)methoxy]-1,3-propanediol, 2,8-diamino-1,9-dihydro-9-[[1-(hydroxymethyl)-2-phenoxyethoxy)methyl]-6*H*-purin-6-one and 2-[(2-amino-8-bromo-6-hydroxy-9*H*-purin-9-yl)methoxy]-1,3-propanediol. The latter compound is not only useful pharmacologically but is also useful as an intermediate for preparing certain compounds of the present invention.

Some of the compounds of formula *1* may be prepared directly according to the following scheme:

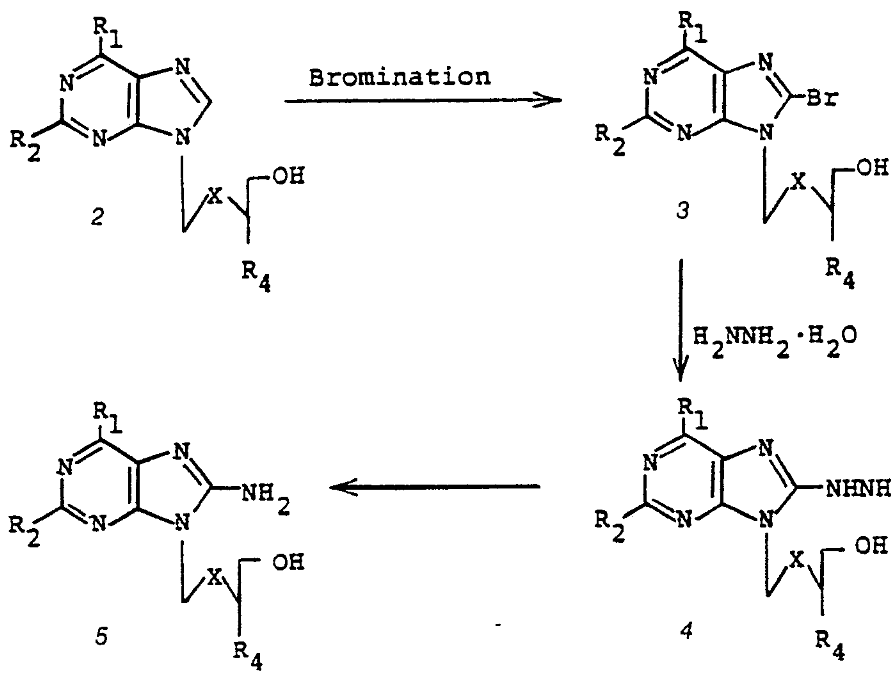


2 3 4 5

The compounds of formula *2* above where $R_1$=OH, $R_2$=$NH_2$, X=O, $R_4$=H or $CH_2OH$ may be prepared according to British Patent Specification 1,567,671 or J. C. Martin, et al, in J. Med. Chem. *26*, 759 (1983). The remainder of the compounds of formula *2* above, used as starting materials and final products are prepared according to the following schemes 1 and 2. They are also included in the claimed subject matter of the first and second divisionals, namely European Patent Applications Nos. 0249247 and 0249248. Treatment of a compound of formula *2* with N-bromosuccinimide in acetic acid, DMF or methanol produces a compound of formula *3* which when treated with hydrazine hydrate gives the hydrazine of formula *4* or directly the 8-amino derivative of formula *5*. The reaction of the 8-bromo compound with hydrazine may or may not proceed entirely to the 8-amino compound. Thus when then 8-hydrazine compound is obtained, it may be further reacted with Raney nickel to allow the reduction to go to completion and afford the desired 8-amino compound. Compounds of formula *5*, wherein $R_1$, $R_2$, and $R_4$ have been defined according to compounds of formula *1* according to the present invention may be further converted by known methods to provide $R_5$ substituents of formula *1* or, for example, where $R_1$ is OH, converting said compound to a compound of formula *1* where $R_1$ is SH by known means.

The compounds of the present invention and of the formulae *1, 2, 3 4,* and *5,* shown above, may also be prepared by the following schematic sequences of reaction steps as illustrated in Schemes 1 and 2. The numbers in parentheses towards the end of each reaction scheme correspond to the compounds as defined above. A more detailed description of the reaction steps is provided in the Examples.

In the preparation of compounds of the present invention and of the formulae *1* and *5*, there are employed novel intermediates which are the subject of European Patent Application No. 0249249, which is a third divisional of the present application. These are compounds of the formula

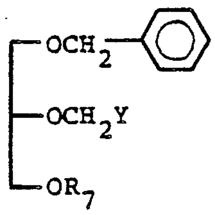

6

wherein Y is acetyloxy or chloro and $R_7$ is alkyl of one to eight carbon atoms, cycloalkyl of five to seven ring members, cycloalkylalkyl, aryl or aralkyl.

SCHEME I

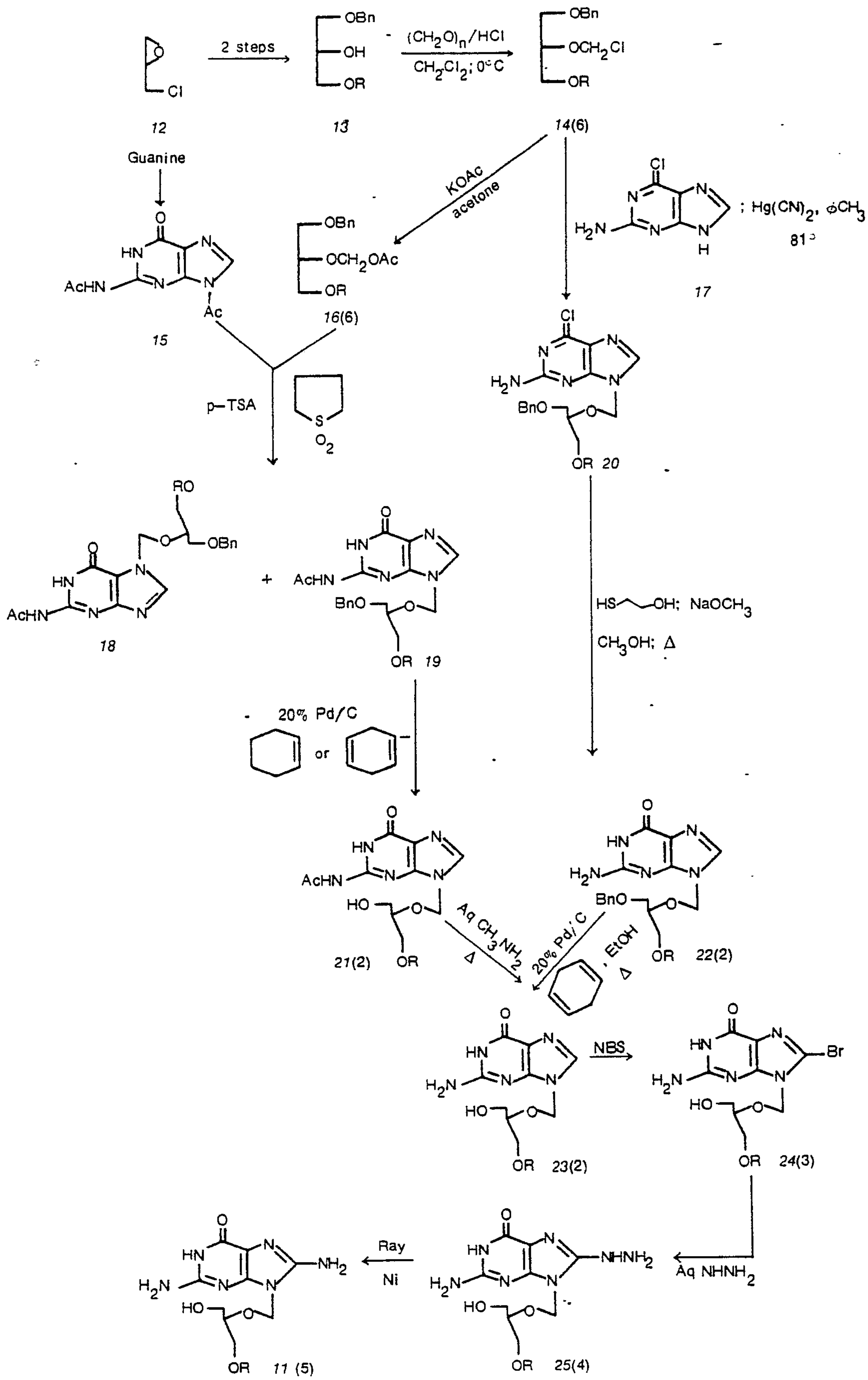
2 steps
$(CH_2O)_n$/HCl
$CH_2Cl_2$; 0°C
12
13
14(6)
Guanine
KOAc
acetone
; $Hg(CN)_2$, $\phi CH_3$
81°
17
15
16(6)
20
p-TSA
18
+
19
HS⌒OH; $NaOCH_3$
$CH_3OH$; Δ
20% Pd/C
or
21(2)
22(2)
Aq $CH_3NH_2$
Δ
20% Pd/C
, EtOH
Δ
NBS
23(2)
24(3)
Ray
Ni
Aq $NHNH_2$
11 (5)
25(4)

Scheme 2

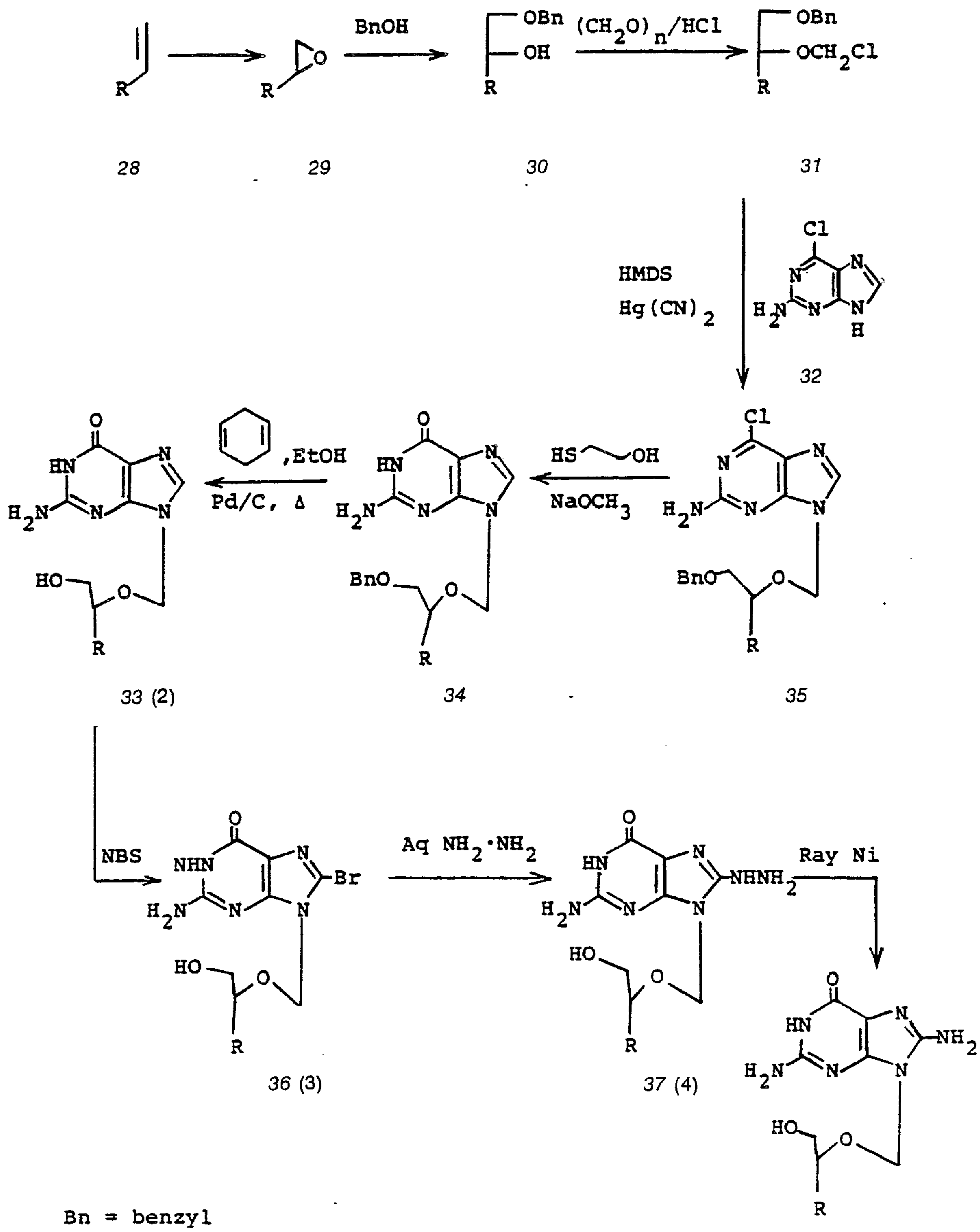


28 29 30 31 32 33 (2) 34 35 36 (3) 37 (4) 26 (5)

Bn = benzyl

The compounds of the present invention have been shown to exhibit significant enzyme inhibition activity and cytotoxic activity. In the purine nucleoside phosphorylase (PNP—4) enzyme assay, total inhibition was achieved at a concentration less than about 300 micromoles on certain compounds. The same compounds also were found by a standard test (Science, *214*, 1137, 1981) to be selectively cytotoxic for T-cells in the presence of 2′-deoxyguanosine at a similar concentration range. For example, 2,8-diamino-9-[(2-hydroxyethoxy)methyl)]-9*H*-purine-6-ol is selectively cytotoxic to T-cell at a concentration of about 30 micromoles in the presence of 10 micromoles of 2′-deoxyguanosine. Similarly, 2-[(2,8-diamino-6-hydroxy-9*H*-purin-9-yl)methoxy]-1,3-propanediol is selectively cytotoxic to T-cell at a concentration of about 7 micromoles in the presence of 10 micromoles of 2′-deoxyguanosine. Both compounds were nontoxic to B-cell in the presence of the same amount of 2′-deoxyguanosine. Since T-cells play a central role in immune response, use of the compounds of the invention is contemplated for the immunoregulation of autoimmune diseases such as rheumatoid arthritis, systemic lupus erythematosus, inflammatory bowel disease, cancer, and viral diseases, transplantation, juvenile diabetes, myasthenia gravis, and multiple sclerosis. The present invention thus includes compositions containing a compound according to the present invention in treating disease such as autoimmune disease characterized by abnormal immune response in warm-blooded animals. According to this aspect of the invention, the properties of the compounds of the invention are utilized by administering to a warm-blooded animal an effective amount of a pharmaceutical composition containing as the active ingredient at least about 0.1 percent by weight, based on the total weight of the composition of at least one such compound of the invention.

Pharmaceutical compositions of the invention can be formulated in any suitable way, preferable with an inert carrier for administration orally, parenterally, ophthalmically, topically, or by suppository.

For example, the compounds of the present invention are formulated into dosage forms such as tablets or syrups by blending with an inert pharmaceutical carrier such as lactose or simple syrup by methods well known in the art. For injectionable dosage forms, they are formulated with vehicles such as water, peanut oil, sesame oil, and the like. In these dosage forms, the active ingredient is from about 0.05 grams to 0.5 grams per dosage unit.

The present invention is further illustrated by way of some of the following examples. Example 1 illustrates the preparation of a known compound which is excluded from the claims. Some of the following examples are directed to compounds according to the present invention, whereas others are directed to compounds claimed in the three divisional applications, namely European Patent Applications Nos. 0249247, 0249248, and 0249249. However, in view of the technical interrelationship between the subject matter claimed in all four applications, the inclusion of those other examples in the present specification assists in a clearer understanding of the methods and concepts involved in the production of the compounds according to the present invention.

## Example 1

2-Amino-8-bromo-9-[(2-hydroxyethoxy)methyl]-9H-purin-6-ol[a]

N-bromosuccinimide (0.415 g; 2.3 mmol) is added to a solution of acyloguanosine (0.5 g; 2.2 mmole) (prepared according to British Patent 1,567,671) in acetic acid (7 ml) and the mixture stirred at room temperature for 20 hours. The solution is then diluted with water (20 ml) and the precipitated product is filtered, washed, and triturated with hot water to give 0.25 g of white solid, mp>300°C.

## Example 1A

The procedure described in Example 1 is repeated to prepared the following 8-bromo-9-substituted guanines starting from appropriate 9-substituted guanines in each case using acetic acid, methanol or DMF as solvent:

2-amino-8-bromo-9-[[2-(heptyloxy)-1-(hydroxymethyl)ethoxy]methyl]-1,9-dihydro-6*H*-purin-6-one, mp>250°C, dec;

2-amino-8-bromo-9-[[2-(hexyloxy)-1-(hydroxymethyl)ethoxy]methyl]-1,9-dihydro-6*H*-purin-6-one, mp>250°C, dec;

2-amino-8-bromo-9-[[2-(butoxy)-1-(hydroxymethyl)ethoxy]methyl]-9*H*-purin-6-ol, mp>200°C;

2-amino-8-bromo-1,9-dihydro-9-[[1-(hydroxymethyl)-2-(octyloxy)ethoxy]methyl]-6*H*-purin-6-one, mp 223—226°C, dec;

2-amino-8-bromo-9-[[2-(hexyloxy)-1-(hydroxymethyl)ethoxy]methyl]-1,9-dihydro-6*H*-purin-6-one, mp 212—214°C;

2-amino-8-bromo-9-[[2-ethoxy-1-(hydroxymethyl)ethoxy]methyl]-1,9-dihydro-6*H*-purin-6-one, mp 217—219°C, dec;

2-amino-8-bromo-1,9-dihydro-9-[[1-(hydroxymethyl)2-(pentyloxy)ethoxy]methyl]-6*H*-purin-6-one, mp>250°C, dec;

---

[a]The structure of this compound is disclosed in Biochem. Pharm., 30, 3071—3077 (1981) by P. M. Keller, et. al.

2-amino-8-bromo-9-[[2-(cyclohexylmethoxy)-1-(hydroxymethyl)ethoxy]methyl]-1,9-dihydro-6*H*-purin-6-one, mp 210—212°C (dec);

2-amino-8-bromo-1,9-dihydro-9-[[1-(hydroxymethyl)-2-(phenoxy)ethoxy]methyl]-6*H*-purin-6-one, mp 218—219°C, dec;

2-amino-8-bromo-1,9-dihydro-9-[[2-(hydroxy)-1-[(4-methoxyphenoxy)methyl)ethoxy]methyl]-6*H*-purin-6-one, mp 205—210°C, dec;

2-amino-8-bromo-1,9-dihydro-9-[[1-(hydroxymethyl)-2-(4-methylphenoxy)ethoxy]methyl]-6*H*-purin-6-one, mp 207—208°C, dec;

2-amino-8-bromo-1,9-dihydro-9-[[2-(4-chlorophenoxy)-1-(hydroxymethyl)ethoxy]methyl]-6*H*-purin-6-one, and

2-amino-8-bromo-1,9-dihydro-9-[[[1-(hydroxymethyl)nonyl]oxy]methyl]-6*H*-purin-6-one, mp 211—212°C, dec.

Example 2

2,8-Diamino-9-[(2-hydroxyethoxy)methyl)]-9H-purin-6-ol

The crude 2-amino-8-bromo-9-[(2-hydroxyethoxy)methyl]-9H-purin-6-ol from acycloguanosine (3.17 g; 0.14 mol) is suspended in water (10 ml) and 97% hydrazine (4 ml) is added to the mixture. The mixture is refluxed for 48 hours, cooled and filtered to give a white solid (1.6 g) which is triturated with hot water (75 ml) to give the analytical sample (1.5 g), mp>300°C dec.

Example 3

2-[(2-Amino-8-bromo-6-hydroxy-9H-purin-9-yl)methoxy]-1,3-propanediol

N-bromosuccinimide (0.375 g; 2.1 mmol) is added to a solution of 9′-[(1,3-dihydroxy-2-propoxy)methyl]guanine (0.5 g; 1.9 mmole) [prepared according to J. C. Martin; C. A. Dvorak, D. F. Smee, T. R. Matthews, and J. P. H. Verheyden, J. Med. Chem. *26*, 759—761 (1983)] in acetic acid (7 ml). The suspension is stirred for 1.5 hours at room temperature and then diluted with water (60 ml). The aqueous solution is concentrated and the residue is recrystallized from water to give 0.44 g of the product; mp>300°C dec.

Example 4

2-[(2,8-Diamino-6-hydroxy-9H-purin-9-yl)methoxy]-1,3-propanediol

A mixture of 2-[(2-amino-8-bromo-6-hydroxy-9H-purin-9-yl)methoxy]-1,3-propanediol (13.7 g; 41 mmole) and 97% hydrazine (6.07 ml) in water (300 ml) is heated to reflux for 48 hours. At the end of this time, the solution is cooled and filtered to give 9.15 g of crude solid. The crude product is suspended in water (120 ml) and Raney nickel (9 g) is added. The mixture is heated at reflux for 6 hours, filtered hot and cooled. The crystals are collected and dried to give 7.15 g of the product, mp>280°C dec.

Example 4A

The procedure described in Example 4 is repeated to prepare the following 8-amino-9-substituted guanines starting from appropriate 8-bromo-9-substituted guanines in each case using methoxyethanol as a cosolvent as necessary to make a homogeneous reaction mixture:

2,8-diamino-9-[[2-ethoxy-1-(hydroxymethyl)ethoxy]methyl]-9*H*-purin-6-ol, mp >220°C, dec;

2,8-diamino-9-[[2-(hexyloxy)-1-(hydroxymethyl)ethoxy]methyl]-1,9-dihydro-6*H*-purin-6-one, mp >265°C, dec;

2,8-diamino-9-[[2-butoxy-1-(hydroxymethyl)ethoxy]methyl]-9*H*-purin-6-ol, mp >240°C, dec;

2,8-diamino-1,9-dihydro-9-[[1-(hydroxymethyl)-2-(pentyloxy)ethoxy]methyl]-6*H*-purin-6-one, mp 274—277°C, dec;

2,8-diamino-9-[[2-(heptyloxy)-1-(hydroxymethyl)ethoxy]methyl]-1,9-dihydro-6*H*-purin-6-one, mp >260°C, dec;

2,8-diamino-9-[[2-(hexyloxy)-1-(hydroxymethyl)ethoxy]methyl]-1,9-dihydro-6*H*-purin-6-one, mp 260—265°C, dec;

2,8-diamino-9-[[2-(cyclohexylmethoxy)-1-(hydroxymethyl)ethoxy]methyl]-1,9-dihydro-6*H*-purin-6-one, mp 242—247°C, dec;

2,8-diamino-1,9-dihydro-9-[[1-(hydroxymethyl)-2-(octyloxy)ethoxy]methyl]-6*H*-purin-6-one, mp >265°C (dec);

2,8-diamino-1,9-dihydro-9-[[1-(hydroxymethyl)-2-(phenoxy)ethoxy]methyl]-6*H*-purin-6-one, mp 265—271°C, dec;

2,8-diamino-1,9-dihydro-9-[[2-hydroxy-1-[(4-methoxyphenoxy)methyl]ethoxy]methyl]-6*H*-purin-6-one;

2,8-diamino-1,9-dihydro-9-[[1-hydroxymethyl)-2-(4-methylphenoxy)ethoxy]methyl]-6*H*-purin-6-one; mp >250°C, dec;

2,8-diamino-1,9-dihydro-9-[[2-(4-chlorophenoxy)-1-(hydroxymethyl)ethoxy]methyl]-6*H*-purin-6-one, and

2,8-diamino-1,9-dihydro-9-[[[1-(hydroxymethyl)nonyl]oxy]methyl]-6*H*-purin-6-one.

Example 5

9-[[2-Benzyloxy-1-(benzyloxymethyl)-ethoxy]methyl]-8-hydrazine-guanine

A mixture of 9-[[2-benzyloxy-1-(benzyloxymethyl)-ethoxy]methyl]guanine (2.1 g); [prepared according to K. K. Ogilvie, V. O. Cheriyan, B. K. Radatus, K. O. Smith, K. S. Galloway, and W. L. Kennell, Can J. Chem, *60,* 3005 (1982)] and N-bromosuccinimide (0.94 g) in acetic acid (21 ml) is stirred overnight and then is diluted with water and extracted with chloroform. The chloroform extract is dried and concentrated to give 2.3 g of yellow oil. The crude oil is suspended in ethanol (100 ml) and treated with 95% hydrazine. The solution is heated to reflux for 24 hours. The reaction mixture is then cooled and the product (0.75 g) filtered and dried, mp >210°C dec.

Example 6

8-Amino-9-[[2-benzyloxy-1-(benzyloxymethyl)-ethoxy]methyl]-guanine

A mixture of 9-[[2-benzyloxy-1-(benzyloxymethyl)-ethoxy]methyl]-8-hydrazine-guanine (0.45 g; 0.98 mmol), water (40 ml), ethanol (40 ml), ammonium hydroxide (20 ml) and Raney nickel (1 g) is heated to reflux for 24 hours. The catalyst is filtered off and the filtrate concentrated to a solid which is recrystallized from ethanol to give 0.16 g of analytical sample, mp 255—260°C dec.

Example 7

N-[9-[[1-(Butoxymethyl)-2-(phenylmethoxy)ethoxy]methyl]-6-hydroxy-9H-purin-2-yl]acetamide

Dry HCl (g) is bubbled into a stirred mixture of paraformaldehyde (1.45 g, 0.048 mol) and 1-butoxy-3-(phenylmethoxy)-2-propanol (5.0 g, 0.021 mol) in methylene chloride (57 ml) at 0°C until all the solid is dissolved. The resulting solution is stored at 0°C for 16 hours, dried over $MgSO_4$, and then evaporated to give chloromethyl glycerol ether as a very unstable clear oil. The clear oil is then added dropwise to a stirred mixture of potassium acetate (5.0 g, 0.051 mol) in acetone (60 ml). The mixture is stirred for 16 hours at room temperature and then filtered and evaporated. The residual oil is dissolved in toluene, washed with saturated $NaHCO_3$ and water, dried and evaporated to give the acetoxy derivative as an oil (5.6 g) which is immediately used for condensation with diacetylguanine.

A mixture of diacetylguanine (4.6 g, 0.0195 mol) and crude acetoxy derivative from above (5.6 g), p-toluene sulfonic acid (43 mg) and sulfolane (5 ml) is heated to 95°C under nitrogen atmosphere for 72 hours. At 24 hours and 48 hours, additional amounts of p-toluene sulfonic acid (20 mg each) are added. After 72 hours, the mixture is cooled, diluted with toluene and filtered. The filtrate is concentrated, chromatographed, and recrystallized from toluene to provide the desired product (1.33 g), mp 139—141°C.

Example 8

The procedure described in Example 7 is repeated to prepare the following guanine-2-acetamide derivatives, starting from diacetylguanine and appropriate 1-(alkoxy or alkyl or substituted phenoxy)-3-(phenylmethoxy-2-propanols in each case.

N-[6,9-dihydro-9-[[1-[(octyloxy)methyl]-2-(phenylmethoxy)ethoxy]methyl]-6-oxo-1*H*-purin-2-yl]-acetamide, mp 127—132°C;

N-[6,9-dihydro-6-oxo-9-[[1-[(phenoxymethyl)-2-(phenylmethoxy)ethoxy]methyl]-1*H*-purin-2-yl]-acetamide, mp 144—146°C, and

N-[9-[[1-(ethoxymethyl)-2-(phenylmethoxy)ethoxy]methyl]-6,9-dihydro-6-oxo-1*H*-purin-2-yl]-acetamide, mp 131—133°C, dec.

Example 9

2-Amino-9-[[2-butoxy-1-(hydroxymethyl)ethoxy]methyl]-9H-purin-6-ol

A mixture of N-[9-[[1-(butoxymethyl)-2-(phenylmethoxy)ethoxy]methyl]-6-hydroxy-9*H*-purin-2-yl]acetamide (1.15 g, 25.9 mmol), 20% palladium on carbon (0.2 g), cyclohexene (20 ml), and ethanol (10 ml) is heated at reflux under $N_2$. After 8 and 20 hours, additional amounts of catalyst (0.1 g) are added. After 36 hours, the solution is cooled, filtered through celite, and the filter cake is washed with DMF/ethanol. The filtrates are combined, refiltered and concentrated. The residue is mixed with aq. methyl amine (20 ml) and the mixture is heated at reflux for two hours, filtered and concentrated. The residue is recrystallized from water to give the desired product (0.7 g), mp 208—211°C.

Example 10

The procedure described in Example 9 is repeated to prepare the following 9-substituted guanine derivatives, starting from N-[9-substituted-6-hydroxy-9*H*-purin-2-yl]acetamides in each case. Cyclohexene and cyclohexadiene can either be used in the transfer hydrogenation reaction:

2-amino-9-[[2-ethoxy-1-(hydroxymethyl)ethoxy]methyl]-1,9-dihydro-6*H*-purin-6-one, mp 206—209°C;

2-amino-1,9-dihydro-9-[[1-(hydroxymethyl)-2-phenoxyethoxy]methyl]-6*H*-purin-6-one, mp 195—198°C, and

2-amino-1,9-dihydro-9-[[1-(hydroxymethyl)-2-(octyloxy)ethoxy]methyl]-6*H*-purin-6-one, mp 227—230°C.

Example 11

2-Amino-9-[[1-[(heptyloxy)methyl]-2-[phenylmethoxy)ethoxy]methyl]-1,9-dihydro-6H-purin-6-one

A mixture of 2-amino-6-chloropurine (Aldrich Chemical Co.) 11.2 g, 0.066 mol) hexamethyldisilazane (160 ml), and ammonium sulfate (1.09 g) is refluxed for 2.5 hours and then cooled, concentrated and pumped to dryness. The residue is dissolved in dry toluene (210 ml) and is treated with $Hg(CN)_2$. The mixture is heated to 80°C and a solution of 2-(chloromethoxy)-1-(heptyloxy-3-(phenylmethoxy)propane (prepared from 1-heptyloxy)-3-(phenylmethoxy)-2-propanol (19 g, 0.068 mol), paraformaldehyde (4 g) and dry HCl (g) in $CH_2Cl_2$ (160 ml) as described in the first part of Example 7, in toluene (210 ml) is added to the solution and heated to 80—85°C for 2.5 hours. The mixture is cooled, concentrated, and diluted with $CH_2Cl_2$ (1.0 L) and is allowed to stand overnight. The $CH_2Cl_2$ solution is filtered, washed with 30% KI, 10% potassium carbonate solution and water. The organic layer is dried and concentrated. The residue is chromatographed over silica gel column using a high pressure liquid chromatographic instrument (Waters Prep 500). The column is eluted with ethyl acetate and hexane (1:1) to give the condensation product (i.e., chloropurine derivative) (6.45 g) which is hydrolysed as follows.

A mixture of the above chloropurine derivative (6.42 g, 0.0139 mol) methanol (150 ml) and sodium methoxide (3 g, 0.056 mol) is treated with mercaptoethanol (4.4 ml) and water (0.26 ml). The mixture is then heated to reflux under nitrogen for two hours and then an additional amount of sodium methoxide (1.9 g) is added. The reaction mixture is heated to reflux for an additional 4.0 hours, cooled, and concentrated to about 50 ml. The concentrate is diluted with water (120 ml) and the solution is acidified to pH 6.0. The solid precipitate is filtered, washed with water, and dried. The crude product is then recrystallized from methanol/water to give an analytical sample (4.25 g), mp 185—187°C.

Example 12

The procedure described in Example 11 is repeated to prepare the following 9-substituted guanines starting from 2-amino-6-chloropurine and appropriate 1-(alkoxy or substituted phenoxy or alkyl)-3-(phenylmethoxy)-2-propanols in each case:

2-amino-9-[1-[(cyclohexylmethoxy)methyl]-2-(phenylmethoxy)ethoxy]methyl]-1,9-dihydro-6*H*-purin-6-one, mp 198—201°C;

2-amino-9-[1-[(hexyloxy)methyl]-2-(phenylmethoxy)ethoxy]methyl]-1,9-dihydro-6*H*-purin-6-one, mp 192—194°C;

2-amino-1,9-dihydro-9-[1-[(pentyloxy)methyl]-2-(phenylmethoxy)ethoxy]methyl]-6*H*-purin-6-one, mp 192—194°C;

2-amino-1,9-dihydro-9-[1-[(octyloxy)methyl]-2-(phenylmethoxy)ethoxy]methyl]-6*H*-purin-6-one, mp 184—186°C;

2-amino-1,9-dihydro-9-[1-(phenoxy)methyl)-2-(phenylmethoxy)ethoxy]methyl]-6*H*-purin-6-one;

2-amino-1,9-dihydro-9-[[[1-[(phenylmethoxy)methyl]hexyl]oxy]methyl-6*H*-purin-6-one, mp 206—208°C;

2-amino-1,9-dihydro-9-[[[1-[(phenylmethoxy)methyl]-nonyl]oxy]methyl-6*H*-purin-6-one, mp 205—207°C;

2-amino-9-[1-[(4-chlorophenoxy)methyl]-2-[phenylmethoxy]ethoxy]methyl]-1,9-dihydro-6*H*-purin-6-one, mp >210°C;

2-amino-1,9-dihydro-9-[1-[(4-methoxyphenoxy)methyl]-2-[phenylmethoxy)ethoxy]methyl]-6*H*-purin-6-one, mp 150—156°C, and;

2-amino-1,9-dihydro-9-[1-[(4-methylphenoxy)methyl]-2-(phenylmethoxy)ethoxy]methyl]-6*H*-purin-6-one, mp 198—200°C.

Example 13

2-Amino-9-[[2-(heptyloxy)-1-(hydroxymethyl)ethoxy]methyl]-1,9-dihydro-6H-purin-6-one

A mixture of 2-amino-9-[1-[(heptyloxy)methyl]-2-(phenylmethoxy)ethoxy]methyl]-1,9-dihydro-6*H*-purin-6-one (3.9 g, 8.97 mmol), ethanol (200 ml), cyclohexadiene (87 ml, 92.3 mmol), and 20% palladium on charcoal (1.5 g) is heated to reflux under nitrogen atmosphere. After seven hours an additional amount of 20% palladium on charcoal (0.5 g) is added and the mixture is heated to reflux for a total of 18 hours. The mixture is filtered hot and then allowed to cool. The solid formed is collected and dried to give the desired purine (1.95 g), mp 224—225°C.

Example 14

The procedure described in Example 13 is repeated to prepare the following 9-substituted guanines starting from appropriate phenylmethoxy derivatives described in Example 11 and 12.

2-amino-1,9-dihydro-9-[[[1-(hydroxymethyl)hexyl]oxy]methyl]-6*H*-purin-6-one, mp 228—229°C;

2-amino-1,9-dihydro-9-[[[1-(hydroxymethyl)nonyl]oxy]methyl]-6*H*-purin-6-one, mp >250°C, dec)

2-amino-9-[[2-(ethoxy)-1-(hydroxymethyl)ethoxy]methyl]-1,9-dihydro-6*H*-purin-6-one, mp 206—209°C-

2-amino-9-[[2-(butoxy)-1-(hydroxymethyl)ethoxy]methyl-9*H*-purin-6-ol, mp 208—211°C;

2-amino-9-[[2-(hexyloxy)-1-(hydroxymethyl)ethoxy]methyl]-1,9-dihydro-6*H*-purin-6-one;

2-amino-1,9-dihydro-9-[[1-(hydroxymethyl)-2-(penyloxy)ethoxy]methyl]-6*H*-purin-6-one, mp 218—220°C;

2-amino-1,9-dihydro-9-[[1-(hydroxymethyl)-2-[2-octyloxy)ethoxy]methyl]-6*H*-purin-6-one, mp 227—230°C;

2-amino-9-[[2-(cyclohexylmethoxy)-1-(hydroxymethyl)ethoxy]methyl]-1,9-dihydro-6*H*-purine-6-one, mp >260°C, dec;

2-amino-1,9-dihydro-9-[[1-(hydroxymethyl)-2-phenoxyethoxy]methyl]-6*H*-purin-6-one, mp 195—198°C;

2-amino-1,9-dihydro--9-[[1-(hydroxymethyl)-2-(4-methylphenoxy)ethoxy]methyl]-6*H*-purin-6-one, mp 206—208°C;

2-amino-1,9-dihydro--9-[[2-(hydroxy-1-[(4-methoxyphenoxy)methyl]ethoxy]methyl]-6*H*-purin-6-one, mp 210—217°C, dec, and

2-amino-9-[[2-(4-chlorophenoxy)-1-(hydroxymethyl)ethoxy]methyl]-1,9-dihydro-6*H*-purin-6-one.

## Synthesis of starting materials

### Example A

1-Butoxy-3-(phenylmethoxy)-2-propanol

n-Butanol (2.5 ml, 27 mmol) is added to a suspension of sodium of sodium hydride (50% in mineral oil; 1.3 g, 27 mmol) in DMF (5 ml) and the mixture is then heated to 80°C for 1.0 hours when all the sodium hydride is consumed. A solution of 2,3-epoxypropyl benzyl ether (benzyl glycidylether*) (4.52 g, 27 mmol) in DMF (5 ml) is added slowly to the n-butoxide solution. The mixture is then heated to 80°C for 16 hours, diluted with water and extracted with ether. The ether layer is dried and concentrated to give an oil which is distilled to provide the desired product (3.1 g), bp 125—130°C/(0.8—0.5 mmHg) $1 \times 10^2$—$7 \times 10^2 N/m^2$.

### Example B

The procedure described in Example A is repeated to prepare the following 1-alkoxy or aryloxy-3-(phenylmethoxy)-2-propanols, starting from appropriate alkanols or phenols in each case.

1-(ethoxy)-3-(phenylmethoxy)-2-propanol, bp 92—99°C/(0.25—0.3 mmHg) $0.33 \times 10^2$—$0.4 \times 10^2 N/m^2$;

1-(pentyloxy)-3-(phenylmethoxy)-2-propanol, bp 115—118°C/10.3 mmHg) $0.4 \times 10^2 N/m^2$;

1-(hexyloxy)-3-(phenylmethoxy)-2-propanol, bp 123—125°C/(0.12 mmHg) $0.16 \times 10^2 N/m^2$-

1-(heptyloxy)-3-(phenylmethoxy)-2-propanol, bp 141°C/(0.36 mmHg) $0.48 \times 10^2 N/m^2$, and

1-(octyloxy)-3-(phenylmethoxy)-2-propanol, bp 150—155°C/(0.7 mmHg) $0.9 \times 10^2 N/m^2$.

### Example C

1-(Phenylmethoxy)-2-decanol

Benzyl alcohol (108 g, 1.0 mol) is added to a suspension of 50% sodium hydride-mineral oil (48 g, 1.0 mol) in DMF (200 ml) at room temperature. The mixture is then heated to 80°C for two hours. A solution of 1,2-epoxydecane** (85 ml) in DMF (50 ml) is added slowly to the sodium salt over 30 minutes and the mixture is then heated at 80°C for 20 hours. The reaction mixture is cooled, diluted with water, neutralized with acetic acid, and extracted with ether. The ether extract is concentrated to give an oil which is distilled to give the desired product (131 g), bp 178—180°C/(4 mmHg) $5 \times 10^2 N/m^2$.

### Example D

The procedue described in Example C is repeated to prepare the following 1-(phenylmethoxy)-alkanols, starting from appropriate 1,2-epoxides in each case.

1-(cyclohexylmethoxy)-3-(phenylmethoxy)-2-propanol, bp 136—139°C/(0.24—0.22 mmHg) $0.32 \times 10^2$—$0.29 \times 10^2 N/m^2$;

1-(phenylmethoxy)-2-heptanol, bp 125—130°C/(3—5 mmHg) $4 \times 10^2$—$7 \times 10^2 N/m^2$;

1-(phenoxy)-3-(phenylmethoxy)-2-propanol, bp 148—157°C/(0.32 mmHg) $0.43 \times 10^2 N/m^2$;

1-(4-methylphenoxy)-3-(phenylmethoxy)-2-propanol, bp 194°C/(2 mmHg) $3 \times 10^2 N/m^2$;

1-(4-methoxyphenoxy)-3-(phenylmethoxy)-2-propanol, bp 175—184°C/(0.4 mmHg) $0.5 \times 10^2 N/m^2$, and

1-(4-chlorophenoxy)-3-(phenylmethoxy)-2-propanol, bp 188—190°C/(1.2—1.3 mmHg) $1.6 \times 10^2$—$1.7 \times 10^2 N/m^2$.

---

*Benzyl glycidyl ether is prepared according to the published procedure (J. R. Bacon and M. J. Collis, Chem. and Ind., *1971*, 930) or purchased from commercial source.

**Purchased from Aldrich Chemical Co. Other epoxides are either purchased or prepared from olefin or epichlorohydrine as the case may be.

**Claims for the Contracting States: BE CH DE FR GB IT LI LU NL SE**

1. A compound having the following general formula:

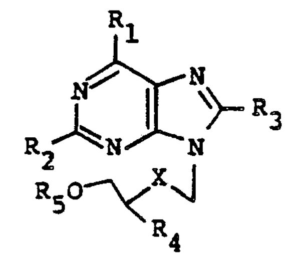

or a tautomeric isomer thereof, or a pharmaceutically acceptable base salt or acid addition salt thereof, wherein:

$R_1$ is —OH or —SH;
$R_2$ is a hydrogen atom or —NHR in which R is hydrogen or —$COR_6$;
$R_6$ is a $C_{1-4}$ alkyl radical, an aryl radical or an aralkyl radical;
$R_3$ is a bromine atom or NHR where R is hydrogen or $COR_6$;
X is an oxygen or a sulphur atom;
$R_4$ is a hydrogen atom or —$CH_2OR_5$;
$R_5$ is hydrogen, a $C_{1-8}$ alkyl radical, an aralkyl radical, an aryl radical, —$COR_6$ or

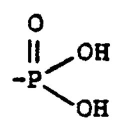 ;

the term "aryl" relates to phenyl rings which are unsubstituted or substituted by halo, alkyl of from 1 to 4 carbon atoms or alkoxy of from 1 to 4 carbon atoms; and

the term "aralkyl" relates to an "aryl" radical, as defined above, attached to an alkyl group of from 1 to 4 carbon atoms;

with the proviso that when $R_1$ is —OH, $R_2$ is —$NH_2$, $R_3$ is a bromine atom and $R_5$ is a hydrogen atom, $R_4$ is not a hydrogen atom.

2. A compound according to Claim 1, or a tautomeric isomer thereof or a pharmaceutically acceptable base or acid addition salt thereof, wherein: $R_6$ is a $C_{1-4}$ alkyl radical or a phenyl radical; $R_3$ is bromine or $NH_2$; and $R_5$ is a hydrogen atom, a $C_{1-8}$ radical, a phenyl radical, or a benzyl radical.

3. A compound according to Claim 1 or 2, wherein: $R_1$ is —OH; $R_2$ is hydrogen or —$NH_2$; $R_3$ is bromine or —$NH_2$; X is an oxygen atom; $R_4$ is a hydrogen atom or —$CH_2OR_5$; and $R_5$ is a hydrogen atom.

4. A compound according to Claim 1, and being
2,8-diamino-9-[(2-hydroxyethoxy)methyl]-9*H*-purin-6-ol;
2-[(2,8-diamino-6-hydroxy-9*H*-purin-9-yl)methoxy]-1,3-propanediol;
2-[(2-amino-8-bromo-6-hydroxy-9*H*-purin-9-yl)methoxy]-1,3-propanediol;
2,8-diamino-1,9-dihydro-9-[[1-(hydroxymethyl)-2-phenoxyethoxy)methyl]-6*H*-purin-6-one.

5. A pharmaceutical composition comprising a compound according to any preceding claim, in admixture with a pharmaceutically acceptable carrier or diluent.

**Claims for the Contracting State: AT**

1. A process for preparing a compound having the following general formula:

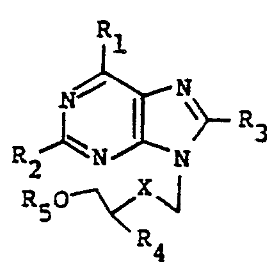

or a tautomeric isomer thereof, or a pharmaceutically acceptable base salt or acid addition salt thereof, wherein:

$R_1$ is —OH or —SH;

$R_2$ is a hydrogen atom or —NHR in which R is hydrogen or —$COR_6$;

$R_6$ is a $C_{1-4}$ alkyl radical, an aryl radical or an aralkyl radical;

$R_3$ is a bromine atom or NHR where R is hydrogen or $COR_6$;

X is an oxygen or a sulphur atom;

$R_4$ is a hydrogen atom or —$CH_2OR_5$;

$R_5$ is hydrogen, a $C_{1-8}$ alkyl radical, an aralkyl radical, an aryl radical, —$COR_6$ or

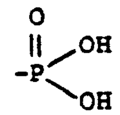
;

the term "aryl" relates to phenyl rings which are unsubstituted or substituted by halo, alkyl of from 1 to 4 carbon atoms or alkoxy of from 1 to 4 carbon atoms; and

the term "aralkyl" relates to an "aryl" radical, as defined above, attached to an alkyl group of from 1 to 4 carbon atoms;

with the proviso that when $R_1$ is —OH, $R_2$ is —$NH_2$, $R_3$ is a bromine atom and $R_5$ is a hydrogen atom, $R_4$ is not a hydrogen atom

which process comprises brominating a compound of the formula

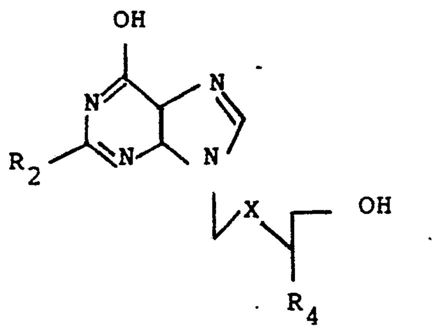

to give the 8-substituted bromo compound; optionally treating the 8-bromo compound with hydrazine and reducing the 8-hydrazine compound to the 8-amino compound; optionally converting the 8-amino compound to the 8-$NHCOR_6$ compound; optionally converting the 8-bromo, 8-amino or 8-$NHCOR_6$ compound to the compound wherein $R_1$ is —SH, and/or $R_5$ is as defined above: and optionally forming the pharmaceutically acceptable base or acid addition salt.

2. A process according to Claim 1, wherein: $R_6$ is a $C_{1-4}$ alkyl radical or a phenyl radical; $R_3$ is bromine or $NH_2$; and $R_5$ is a hydrogen atom, a $C_{1-8}$ radical, a phenyl radical, or a benzyl radical.

3. A process according to Claim 1 or 2, wherein: $R_1$ is —OH; $R_2$ is hydrogen or —$NH_2$; $R_3$ is bromine or —$NH_2$; X is an oxygen atom; $R_4$ is a hydrogen atom or —$CH_2OR_5$; and $R_5$ is a hydrogen atom.

4. A process according to Claim 1, for preparing a compound having the name

2,8-diamino-9-[(2-hydroxyethoxy)methyl]-9*H*-purin-6-ol;

2-[(2,8-diamino-6-hydroxy-9*H*-purin-9-yl)methoxy]-1,3-propanediol;

2-[(2-amino-8-bromo-6-hydroxy-9*H*-purin-9-yl)methoxy]-1,3-propanediol;

2,8-diamino-1,9-dihydro-9-[[1-(hydroxymethyl)-2-phenoxyethoxy)methyl]-6*H*-purin-6-one.

5. A process for preparing a pharmaceutical composition, which process comprises combining a compound prepared by a process as claimed in any preceding claim with a pharmaceutically acceptable carrier or diluent.

**Patentansprüche für die Vertragsstaaten: BE CH DE FR GB IT LI LU NL SE**

1. Verbindung mit der folgenden allgemeinen Formel:

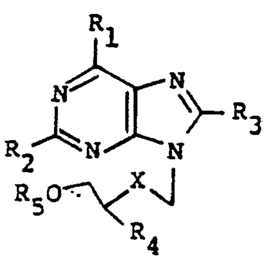

oder ein tautomeres Isomeres davon, oder ein pharmazeutisch akzeptables Basensalz oder Säureadditionssalzes davon, worin:

$R_1$ —OH oder —SH bedeutet;

$R_2$ ein Wasserstoffatom oder —NHR darstellt, worin R für Wasserstoff oder —$COR_6$ steht;

$R_6$ ein $C_{1-4}$-Alkylrest, ein Arylrest oder ein Aralkylrest ist;

$R_3$ ein Bromatom oder NHR bedeutet, worin R für Wasserstoff oder $COR_6$ steht;

X ein Sauerstoff- oder ein Schwefelatom darstellt;

$R_4$ ein Wasserstoffatom oder —$CH_2OR_5$ ist;

$R_5$ Wasserstoff, einen $C_{1-8}$-Alkylrest, einen Aralkylrest, einen Arylrest, —$COR_6$ oder

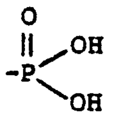

bedeutet;

der Ausdruck "Aryl" Phenylringe, die unsubstituiert sind oder substituiert sind durch Halogen, Alkyl mit 1 bis 4 Kohlenstoffatomen oder Alkoxy mit 1 bis 4 Kohlenstoffatomen bedeutet; und

der Ausdruck "Aralkyl" einen "Aryl"-rest, wie oben definiert, der an einer Alkylgruppe mit 1 bis 4 Kohlenstoffatomen hängt, bedeutet;

mit der Maßgabe, daß, wenn $R_1$ für —OH, $R_2$ für —$NH_2$, $R_3$ für ein Bromatom und $R_5$ für ein Wasserstoffatom steht, $R_4$ kein Wasserstoffatom ist.

2. Verbindung nach Anspruch 1 oder ein tautomeres Isomeres davon oder ein pharmazeutisch akzeptables Basen- oder Säureadditionssalz davon, worin: $R_6$ ein $C_{1-4}$-Alkylrest oder ein Phenylrest ist; $R_3$ für Brom oder $NH_2$ steht; und $R_5$ ein Wasserstoffatom, ein $C_{1-8}$-Rest, ein Phenylrest oder ein Benzylrest ist.

3. Verbindung nach Anspruch 1 oder 2, worin: $R_1$ für OH steht; $R_2$ Wasserstoff oder —$NH_2$ bedeutet; $R_3$ Brom oder —$NH_2$ darstellt; X für ein Sauerstoffatom steht; $R_4$ ein Wasserstoffatom oder —$CH_2OR_5$ bedeutet; und $R_5$ ein Wasserstoffatom darstellt.

4. Verbindung nach Anspruch 1, welche

2,8-Diamino-9-[(2-hydroxyäthoxy)-methyl]-9*H*-purin-6-ol;

2-[(2,8-Diamino-6-hydroxy-9*H*-purin-9-yl)-methoxy]-1,3-propandiol;

2-[(2-Amino-8-brom-6-hydroxy-9*H*-purin-9-yl)methoxy]-1,3-propandiol;

2,8-Diamino-1,9-dihydro-9-[[1-(hydroxymethyl)-2-phenoxyäthoxy]methyl]-6*H*-purin-6-on ist.

5. Pharmazeutische Zusammensetzung, welche eine Verbindung nach einem vorhergehenden Anspruch in Mischung mit einem pharmazeutisch akzeptablen Träger oder Verdünnungsmittel umfaßt.

**Patentansprüche für den Vertragsstaat: AT**

1. Verfahren zur Herstellung einer Verbindung mit der folgenden allgemeinen Formel:

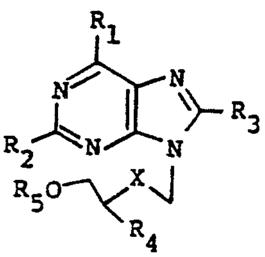

oder eines tautomeren Isomeren davon, oder eines pharmazeutisch akzeptablen Basensalzes oder Säureadditionssalzes davon, worin:

$R_1$ —OH oder —SH bedeutet;

$R_2$ ein Wasserstoffatom oder —NHR darstellt, worin R für Wasserstoff oder —$COR_6$ steht;

$R_6$ ein $C_{1-4}$-Alkylrest, ein Arylrest oder ein Aralkylrest ist;

$R_3$ ein Bromatom oder NHR bedeutet, worin R für Wasserstoff oder $COR_6$ steht;

X ein Sauerstoff- oder ein Schwefelatom darstellt;

$R_4$ ein Wasserstoffatom oder —$CH_2OR_5$ ist;

$R_5$ Wasserstoff, einen $C_{1-8}$-Alkylrest, einen Aralkylrest, einen Arylrest, —$COR_6$ oder

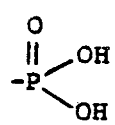

bedeutet;

der Ausdruck "Aryl" Phenylringe, die unsubstituiert sind oder substituiert sind durch Halogen, Alkyl

mit 1 bis 4 Kohlenstoffatomen oder Alkoxy mit 1 bis 4 Kohlenstoffatomen bedeutet; und

der Ausdruck "Aralkyl" einen "Aryl"-rest, wie oben definiert, der an einer Alkylgruppe mit 1 bis 4 Kohlenstoffatomen hängt, bedeutet;

mit der Maßgabe, daß, wenn $R_1$ für —OH, $R_2$ für —$NH_2$, $R_3$ für ein Bromatom und $R_5$ für ein Wasserstoffatom steht, $R_4$ kein Wasserstoffatom ist, welches Verfahren das Bromieren einer Verbindung der Formel

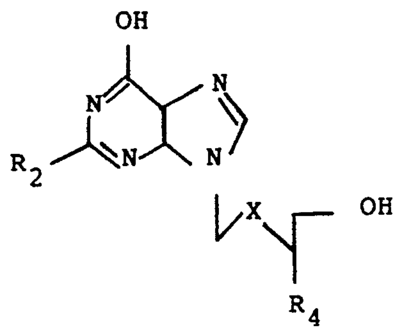

zur Herstellung der 8-substituierten Brom-Verbindung; gegebenenfalls die Behandlung der 8-Brom-Verbindung mit Hydrazin und die Reduktion der 8-Hydrazin-Verbindung zur 8-Amino-Verbindung; gegebenenfalls die Umwandlung der 8-Amino-Verbindung in die 8-$NHCOR_6$-Verbindung; gegebenenfalls die Umwandlung der 8-Bromo-, 8-Amino- oder 8-$NHCOR_6$-Verbindung in die Verbindung, in welcher $R_1$ für —SH steht und/oder $R_5$ der obigen Definition entspricht; und gegebenenfalls die Bildung des pharmazeutisch akzeptablen Basen- oder Säureadditionssalzes umfaßt.

2. Verfahren nach Anspruch 1, worin: $R_6$ ein $C_{1-4}$-Alkylrest oder ein Phenylrest ist; $R_3$ für Brom oder $NH_2$ steht; und $R_5$ ein Wasserstoffatom, ein $C_{1-8}$-Rest, ein Phenylrest oder ein Benzylrest ist.

3. Verfahren nach Anspruch 1 oder 2, worin: $R_1$ für OH steht; $R_2$ Wasserstoff oder —$NH_2$ bedeutet; $R_3$ Brom oder —$NH_2$ darstellt; X für ein Sauerstoffatom steht; $R_4$ ein Wasserstoffatom oder —$CH_2OR_5$ bedeutet; und $R_5$ ein Wasserstoffatom darstellt.

4. Verfahren nach Anspruch 1 zur Herstellung einer Verbindung mit der Bezeichnung

2,8-Diamino-9-[(2-hydroxyäthoxy)-methyl]-9*H*-purin-6-ol;

2-[(2,8-Diamino-6-hydroxy-9*H*-purin-9-yl)methoxy]-1,3-propandiol;

2-[(2-Amino-8-brom-6-hydroxy-9*H*-purin-9-yl)methoxy]-1,3-propandiol;

2,8-Diamino-1,9-dihydro-9-[[1-(hydroxymethyl)-2-phenoxyäthoxy]methyl]-6*H*-purin-6-on.

5. Verfahren zur Herstellung einer pharmazeutischen Zusammensetzung, welches Verfahren die Kombination einer mittels eines Verfahrens, wie in einem vorhergehenden Anspruch beansprucht, hergestellten Verbindung mit einem pharmazeutisch akzeptablen Träger oder Verdünnungsmittel umfaßt.

**Revendications pour les Etats contractants: BE CH DE FR GB IT LI LU NL SE**

1. Un dérivé de formule générale suivante:

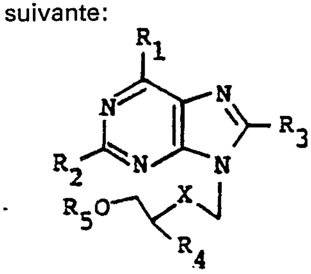

ou un de ses tautomères, ou un sel de base ou un sel d'addition d'acides pharmaceutiquement acceptables de ce dérivé, formule dans laquelle:

$R_1$ représente OH ou SH;

$R_2$ représente un atome d'hydrogène ou NHR dans lequel R représente un atome d'hydrogène ou $COR_6$;

$R_6$ représente un radical alkyle en $C_1$ à $C_4$, un radial aryle ou un radical aralkyle;

$R_3$ représente un atome de brome ou NHR dans lequel R représente un atome d'hydrogène ou $COR_6$;

X représente un atome d'oxygène ou un atome de souffre;

$R_4$ représente un atome d'hydrogène ou le groupe $CH_2OR_5$;

$R_5$ représente un atome d'hydrogène, un radical alkyle en $C_1$ à $C_8$, un radical aralkyle, un radical aryle, $COR_6$ ou:

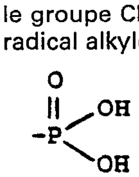

le terme "aryle" signifie des cycles du phényle qui sont non substitués ou substitués par un atome d'halogène, un radical alkyle renfermant de 1 à 4 atomes de carbone ou alkoxy renfermant de 1 à 4 atomes de carbone; et le terme "aralkyle" signifie un radical akyle tel que défini ci-dessus attaché à un groupe alkyle renfermant de 1 à 4 atomes de carbone;

sous réserve que lorsque $R_1$ représente OH, $R_2$ représente $NH_2$, $R_3$ représente un atome de brome et $R_5$ représente un atome d'hydrogène, $R_4$ ne représente pas un atome d'hydrogène.

2. Un dérivé selon la revendication 1, ou un de ses tautomères ou un sel de base ou d'addition d'acides pharmaceutiquement acceptables dans lequel, $R_6$ représente un radical alkyle en $C_1$ à $C_4$ ou un radical phényle; $R_3$ représente un atome de brome ou $NH_2$ et $R_5$ représente un atome d'hydrogène, un radical alkyle en $C_1$ à $C_8$, un radical phényle ou un radical benzyle.

3. Un dérivé selon la revendication 1 ou 2, dans lequel $R_1$ représente OH; $R_2$ représente un atom d'hydrogène ou $NH_2$; $R_3$ représente un atome de brome ou $NH_2$; X représente un atome d'oxygène; $R_4$ représente un atome d'hydrogène ou $CH_2OR_5$ et $R_5$ représente un atome d'hydrogène.

4. Un dérivé selon la revendication 1, qui est le

2,8-diamino-9-[(2-hydroxyéthoxy)méthyl]-9H-purin-6-ol;

2-[(2,8-diamino-6-hydroxy-9H-purin-9-yl)méthoxy]-1,3-propanediol;

2-[(2-amino-8-bromo-6-hydroxy-9H-purin-9-yl)méthoxy]-1,3-propanediol;

2,8-diamino-1,9-dihydro-9-[[(1-(hydroxyméthyl)-2-phénoxyéthoxy]méthyl]-6H-purin-6-one.

5. Une composition pharmaceutique comprenant un composé selon une quelconque des revendications précédentes, associé à un support ou diluant pharmaceutiquement acceptable.

**Revendications pour l'Etat contractant: AT**

1. Un procédé de préparation d'un dérivé de formule générale:

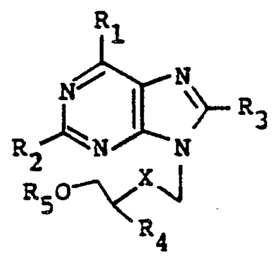

ou d'un de ses tautomères, ou d'un sel de base ou d'addition d'acides pharmaceutiquement acceptables dans lequel:

$R_1$ représente OH ou SH;

$R_2$ représente un atome d'hydrogène ou NHR dans lequel R représente un atome d'hydrogène ou $COR_6$;

$R_6$ représente un radical alkyle en $C_1$ à $C_4$, un radial aryle ou un radical aralkyle;

$R_3$ représente un atome de brome ou NHR dans lequel R représente un atome d'hydrogène ou $COR_6$;

X représente un atome d'oxygène ou un atome de souffre;

$R_4$ représente un atome d'hydrogène ou le groupe $CH_2OR_5$;

$R_5$ représente un atome d'hydrogène, un radical alkyle en $C_1$ à $C_8$, un radical aralkyle, un radical aryle, $COR_6$ ou:

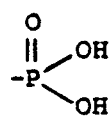

le terme "aryle" signifies des cycles du phényle qui sont non substitués ou substitués par un atome d'halogène, un radical alkyle renfermant de 1 à 4 atomes de carbone ou alkoxy renfermant de 1 à 4 atomes de carbone; et le terme "aralkyle" signifie un radical akyle tel que défini ci-dessus attaché à un groupe alkyle renfermant de 1 à 4 atomes de carbone;

sous réserve que lorsque $R_1$ représente OH, $R_2$ représente $NH_2$, $R_3$ représente un atome de brome et $R_5$ représente un atome d'hydrogène, $R_4$ ne représente pas un atome d'hydrogène, ce procédé comprenant les étapes suivantes:

on brome un dérivé de formule:

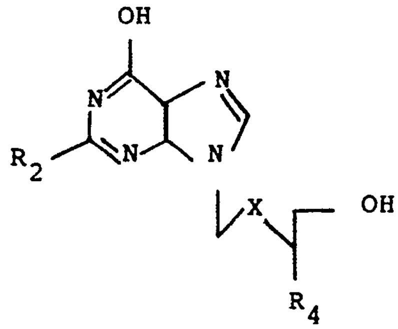

pour obtenir le dérivé bromé en position 8;

éventuellement on traite le dérivé 8-bromo par de l'hydrazine et on réduit le dérivé 8-hydrazine en dérivé 8-amino;

éventuellement on convertit le dérivé 8-amino en dérivé 8-$NHCOR_6$;

éventuellement on convertit les dérivés 8-bromo, 8-amino ou 8-$NHCOR_6$ en dérivés dans lesquels $R_1$ représente SH et/ou $R_5$ est tel que défini ci-dessus;

et éventuellement on forme le sel de base ou d'addition d'acides pharmaceutiquement acceptables.

2. Un procédé selon la revendication 1, dans lequel $R_6$ représente un radical alkyle en $C_1$ à $C_4$ ou un radical phényle, $R_3$ représente un atome de bore ou le groupe $NH_2$ et $R_5$ représente un atome d'hydrogène, un radical en $C_1$ à $C_8$, un radical phényle ou un radical benzyle.

3. Un procédé selon la revendication 1 ou 2 dans lequel:

$R_1$ représente OH;

$R_2$ représente un atom d'hydrogène ou $NH_2$;

$R_3$ représente un atome de brome ou $NH_2$;

X représente un atome d'oxygène;

$R_4$ représente un atome d'hydrogène ou $CH_2OR_5$ et

$R_5$ représente un atome d'hydrogène.

4. Un procédé selon la revendication 1, pour préparer un dérivé désigné sous le nom

2,8-diamino-9-[(2-hydroxyéthoxy)méthyl]-9H-purin-6-ol;

2-[(2,8-diamino-6-hydroxy-9H-purin-9-yl)méthoxy]-1,3-propanediol;

2-[(2-amino-8-bromo-6-hydroxy-9H-purin-9-yl)méthoxy]-1,3-propanediol;

2,8-diamino-1,9-dihydro-9-[[(1-(hydroxyméthyl)-2-phénoxyéthoxy]méthyl]-6H-purin-6-one.

5. Une procédé de préparation d'une composition pharmaceutique, ce procédé consistant à associer un dérivé préparé par un procédé selon l'une quelconque des revendications précédentes à un support ou diluant pharmaceutiquement acceptable.